# EUROPEAN PATENT APPLICATION

(11) **EP 3 613 335 A1**
(43) Date of publication of application: **26.02.2020**
(21) Application number: 18189731.5
(22) Date of filing: 20.08.2018
(51) Int. Cl.: A61B 5/00, A61B 5/11, A61B 5/16, A61B 3/113

(54) **A METHOD AND APPARATUS FOR DETECTING AN EPILEPTIC SEIZURE IN A SUBJECT**

(71) Applicant: Koninklijke Philips N.V., 5656 AE Eindhoven (NL)
(72) Inventor: AARTS, Ronaldus Maria, 5656 AE Eindhoven (NL); JOHNSON, Mark Thomas, 5656 AE Eindhoven (NL); TEN KATE, Warner Rudolph Theophile, 5656 AE Eindhoven (NL); DONGRE, Chaitanya, 5656 AE Eindhoven (NL); VAN DE WOUW, Doortje, 5656 AE Eindhoven (NL)
(74) Representative: de Haan, Poul Erik

(57) **Abstract**

According to an aspect, there is provided an apparatus (2) for detecting an epileptic seizure in a subject, the apparatus (2) comprising a processing unit (4) configured to obtain head movement measurements, wherein the head movement measurements are measurements of movements of a head of the subject; obtain pupil position measurements, wherein the pupil position measurements are measurements of a position of a pupil in a visible part of an eye of the subject; process the head movement measurements relating to a time period to determine if the head moved in the time period; and, if the head moved in the time period, process the head movement measurements and the pupil position measurements for the time period to determine whether the subject experienced a seizure in the time period.

## Description

### FIELD OF THE INVENTION

This disclosure relates to a method and apparatus for detecting an epileptic seizure in a subject.

### BACKGROUND OF THE INVENTION

An epileptic seizure occurs due to (temporary) abnormal activity in the brain. The outward (i.e. visible) signs of a seizure can vary, and can include uncontrolled movement (e.g. jerking), or a loss of awareness or consciousness. It is desirable to be able to monitor a subject to detect the occurrence of seizures (for example to monitor their frequency or severity, including in response to a treatment or medication). The 'gold' standard for assessing the type and severity of an epileptic seizure is by using an electroencephalogram (EEG) in combination with a video recording during a seizure. The EEG monitors the electrical activity in the brain. However, seizures happen randomly, and monitoring a subject using an EEG and video is cumbersome for the subject and expensive. If a subject does not regularly experience a seizure, there is currently no alternative type of monitoring system that can be used unobtrusively during normal life.

### SUMMARY OF THE INVENTION

It is therefore an object to provide an alternative to the use of EEG in combination with a video recording for detecting an epileptic seizure in a subject.

According to a first specific aspect, there is provided an apparatus for detecting an epileptic seizure in a subject, the apparatus comprising a processing unit configured to obtain head movement measurements, wherein the head movement measurements are measurements of movements of a head of the subject; obtain pupil position measurements, wherein the pupil position measurements are measurements of a position of a pupil in a visible part of an eye of the subject; process the head movement measurements relating to a time period to determine if the head moved in the time period; and, if the head moved in the time period, process the head movement measurements and the pupil position measurements for the time period to determine whether the subject experienced a seizure in the time period.

According to a second aspect, there is provided a method of detecting an epileptic seizure in a subject, the method comprising obtaining head movement measurements, wherein the head movement measurements are measurements of movements of a head of the subject; obtaining pupil position measurements, wherein the pupil position measurements are measurements of a position of a pupil in a visible part of an eye of the subject; processing the head movement measurements relating to a time period to determine if the head moved in the time period; and if the head moved in the time period, processing the head movement measurements and the pupil position measurements for the time period to determine whether the subject experienced a seizure in the time period.

According to a third aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram illustrating an apparatus according to an exemplary embodiment;
Fig. 2 illustrates some exemplary pupil positions that can be observed in a subject using a pupil position sensor;
Fig. 3 is a flow chart illustrating a method of detecting an epileptic seizure in a subject according to an exemplary embodiment;
Fig. 4 illustrates exemplary head movements and corresponding pupil positions over time for a subject that is experiencing a seizure;
Fig. 5 illustrates exemplary head movements and corresponding pupil positions over time for a subject that is not experiencing a seizure; and
Fig. 6 illustrates other exemplary head movements and corresponding pupil positions over time for a subject that is not experiencing a seizure.

### DETAILED DESCRIPTION OF EMBODIMENTS

The method and apparatus described herein aim to detect an epileptic seizure in a subject by analysing whether (and if so, how) a pupil in an eye of the subject (or the pupils in both eyes) moves when the head of the subject moves. Thus, measurements indicating movements of the head of the subject are analysed in conjunction with measurements indicating the position of a pupil on a visible part of the eye (or both pupils in both eyes) to determine if the subject is experiencing a seizure.

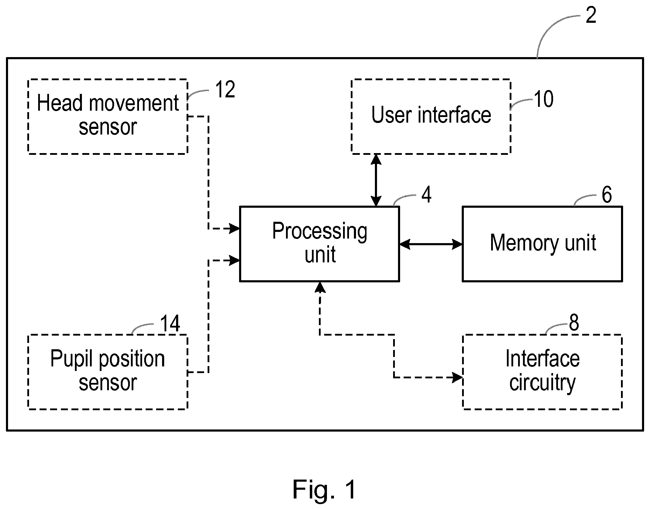

Fig. 1 is a block diagram of an exemplary apparatus 2 for detecting an epileptic seizure in a subject.

The apparatus 2 includes a processing unit 4 that controls the operation of the apparatus 2 and that can be configured to execute or perform the methods described herein. In particular, the processing unit 4 can process measurements of movements of the head of the subject and measurements of the position of a pupil on a visible part of the eye to determine if the subject is experiencing a seizure. The processing unit 4 can be implemented in numerous ways, with software and/or hardware, to perform the various functions described herein. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 4 is connected to a memory unit 6 that can store data, information and/or signals for use by the processing unit 4 in controlling the operation of the apparatus 2 and/or in executing or performing the methods described herein. For example, the memory unit 6 can store measurements of movements of the head of the subject and/or measurements of the position of a pupil on a visible part of the eye prior to processing by the processing unit 4. The memory unit 4 can also or alternatively store intermediate products produced by the processing unit 4 during the processing according to the techniques described herein, and/or the output of the seizure detection (e.g. 'seizure detected', or 'no seizure detected'). In some implementations the memory unit 6 stores computer-readable code that can be executed by the processing unit 4 so that the processing unit 4 performs one or more functions, including the methods described herein. The memory unit 6 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 2 can include interface circuitry 8 for enabling a data connection to and/or data exchange with other devices, including any one or more of servers, databases, user devices, and sensors. The interface circuitry 8 may be used to communicate the outcome of the seizure detection techniques (e.g. 'seizure detected', or 'no seizure detected') to another device, for example to obtain assistance for the subject if a seizure is detected. The connection may be direct or indirect (e.g. via the Internet), and thus the interface circuitry 8 can enable a connection between the apparatus 2 and a network, such as the Internet, via any desirable wired or wireless communication protocol. For example, the interface circuitry 8 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 8 (and thus apparatus 2) may include one or more suitable antennas for transmitting/receiving over a transmission medium (e.g. the air). Alternatively, in the case of a wireless connection, the interface circuitry 8 may include means (e.g. a connector or plug) to enable the interface circuitry 8 to be connected to one or more suitable antennas external to the apparatus 2 for transmitting/receiving over a transmission medium (e.g. the air). The interface circuitry 8 is connected to the processing unit 4.

In some embodiments, the apparatus 2 can include a user interface 10 that includes one or more components that enables a user of apparatus 2 to input information, data and/or commands into the apparatus 2, and/or enables the apparatus 2 to output information or data to the user of the apparatus 2. The user interface 10 can comprise any suitable input component(s), including but not limited to a keyboard, keypad, one or more buttons, switches or dials, a mouse, a track pad, a touchscreen, a stylus, an image capture device, a microphone, etc., and the user interface 10 can comprise any suitable output component(s), including but not limited to a display screen, one or more lights or light elements, one or more loudspeakers, a vibrating element, etc.

As noted above, the processing unit 4 is to process measurements of movements of the head of the subject and measurements of the position of a pupil on a visible part of the eye to determine if the subject is experiencing a seizure. These measurements are obtained by a head movement sensor and a pupil position sensor, and, in some embodiments, the head movement sensor and pupil position sensor are part of the apparatus 2. In other embodiments, the head movement sensor and pupil position sensor are separate from the apparatus 2, and the sensor measurements are communicated to the apparatus 2 for analysis. In other embodiments, one of the head movement sensor and pupil position sensor are part of the apparatus 2, and the other one is separate from the apparatus 2, with the measurements from that sensor being communicated to the apparatus 2.

In Fig. 1, a head movement sensor 12 and pupil position sensor 14 are shown as being part of the apparatus.

The head movement sensor (including the head movement sensor 12 in Fig. 1) can be any type of sensor that can measure the movements of the subject's head, or measure the position and/or orientation of the subject's head (from which measurements of movements can be derived). The head movement sensor 12 can output a signal representing the measured movements over time (for example a signal that comprises a time series of measurement samples of the movement of the head), or a signal representing the measured positions/orientations over time from which the movements can be derived (for example a signal that comprises a time series of measurement samples of the position/orientation of the head). Movements of the subject's head can relate to changes in the orientation of the head (e.g. looking up, looking down, looking straight ahead, looking to the left, looking to the right). In some embodiments, the head movement sensor 12 is one or more accelerometers that are worn on the head and that measure accelerations due to movement of the head, and that measure the direction in which gravity acts. Alternatively the head movement sensor 12 can be one or more gyroscopes that can be worn on the head and that output a signal indicative of the orientation and/or rotation of the head over time. In other embodiments, the head movement sensor 12 can be an image capture device that is positioned to observe the subject's head and capture a video sequence or a series of images, with the video sequence/images being processed or analysed (e.g. by the processing unit 4) to identify the head in the video sequence and the position/orientation of the head over time. In some embodiments, a combination of the above exemplary types of movement sensors can be used. Those skilled in the art will be aware of other types of sensors that can be used to measure the movements of the head of a subject.

The pupil position sensor (including the pupil position sensor 14 in Fig. 1) can be any type of sensor that can measure the position of the pupil in the visible part of an eye of the subject. The pupil position sensor 14 can output a signal representing the measured pupil position over time (for example a signal that comprises a time series of measurement samples of the pupil position at each sampling instant). The pupil position sensor may directly measure the position of the pupil (for example by detecting the pupil and its position) or the pupil position sensor may indirectly measure the position of the pupil (for example by detecting the iris (that is in a fixed relationship with respect to the pupil) and its position) and thereby infer the position of the pupil.

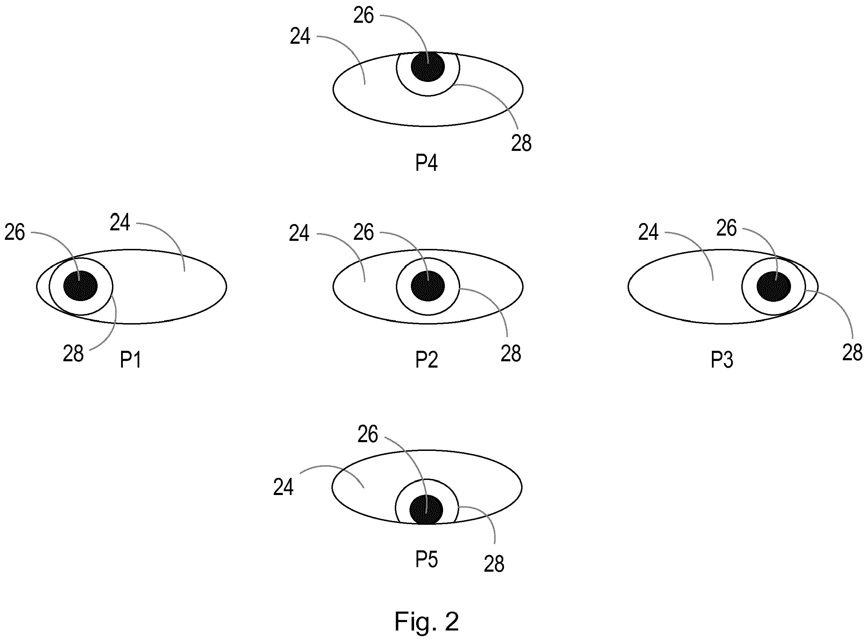

Fig. 2 illustrates some exemplary pupil positions that can be observed in a subject. Five pupil positions are shown and they are 'right', 'central', 'left', 'up' and 'down' (all in the subject's frame of reference - i.e. 'right' refers to the eye of the subject looking to the right). These positions are labelled P1, P2, P3, P4 and P5 respectively. Each pupil position is shown by a representation of a visible part 22 of an eye/eye ball 24. As used herein, the 'visible part' 22 of the eye 24 is the part of the eye ball that is exposed to the air/environment between the eyelids of the subject, and that can be observed externally. Each representation shows a pupil 26 and iris 28 in the appropriate position. It will be appreciated that although five exemplary pupil positions are shown in Fig. 2 and used in the following explanation of the invention, a pupil 26 can be in any visible position between the eyelids.

The position of the pupil 26 can be measured or represented in a number of different ways. In some embodiments, the position of the pupil 26 can be measured or represented in terms of a distance and direction of the pupil 26 from a particular part of the visible part 22 of the eye 24 (e.g. a distance and direction of the pupil 26 from the centre of the visible part 22). In other embodiments, the position of the pupil 26 can be measured or represented in terms of an angle of rotation of the eye 24/pupil 26 and direction of rotation of the pupil 26 from a particular part of the visible part 22 of the eye 24 (e.g. an angle of rotation and a direction of rotation of the pupil 26 from the centre of the visible part 22 of the eye 24). In other embodiments, the position of the pupil 26 can be represented as one of a discrete number of positions (e.g. one of P1-P5 illustrated in Fig. 2), in which can measurements of the position of the pupil 26 can be analysed to classify the position into one of a discrete set of possible positions (in this embodiment it will be appreciated that this classification may be performed by the processing unit 4 rather than the pupil position sensor 14).

In some embodiments, the pupil position sensor 14 is an image capture device that is positioned to observe one or both of the subject's eyes and capture a video sequence or a series of images of the eye(s), with the video sequence/images being processed or analysed (e.g. by the processing unit 4) to identify the position of the pupil 24 in one or both of the eyes of the subject over time. Those skilled in the art will be aware of various processing techniques that can be used to process a video sequence or images to determine a pupil position (e.g. those techniques relating to determining a gaze direction), and further details are not provided herein. The image capture device may be positioned to directly observe one or both of the subject's eyes, or the image capture device may be positioned to observe one or both of the subject's eyes via one or more mirrors and/or lenses.

Those skilled in the art will also be aware of other types of sensors that can be used to measure the position of a pupil in an eye of a subject. For example, a sensor located in or on a contact lens that is worn on the eye of the subject can be used to measure the position of a pupil. Suitable sensors include an accelerometer, a magnetometer and a gyroscope. In another example, the subject can wear a contact lens that serves as a 'tag' for the eye, and the position of the tag (and thus the pupil position) can be monitored by a remote sensor (for example in or on a pair of glasses or a base station located in the environment). As another example, an ultrasound sensor can be used to determine the position of the pupil.

In some embodiments, the positions of both pupils 24 are to be evaluated by the processing unit 4, in which case a second pupil position sensor 14 can be used to provide measurements of the position of the pupil in the other eye, or a video sequence/images of both eyes from a single image capture device can be analysed to determine the positions of both pupils.

The apparatus 2 can be any type of electronic device or computing device. For example the apparatus 2 can be, or be part of, a server, a computer, a laptop, a tablet, a smartphone, a smartwatch, a pendant, a pair of spectacles (glasses), a personal help button (PHB) device, etc. Thus, in some embodiments the apparatus 2 is portable, e.g. in a form that can be carried or worn by the subject, and this may particularly be the case where the apparatus 2 includes the head movement sensor 12 and/or pupil position sensor 14. In some preferred embodiments, the apparatus 2 may include the head movement sensor 12 and pupil position sensor 14 and be in a form that can be worn on the head. Thus, for example, the apparatus 2 can be in the form of a pair of spectacles or a head band.

It will be appreciated that a practical implementation of an apparatus 2 may include additional components to those shown in Fig. 1. For example the apparatus 2 may also include a power supply, such as a battery, or components for enabling the apparatus 2 to be connected to a mains power supply.

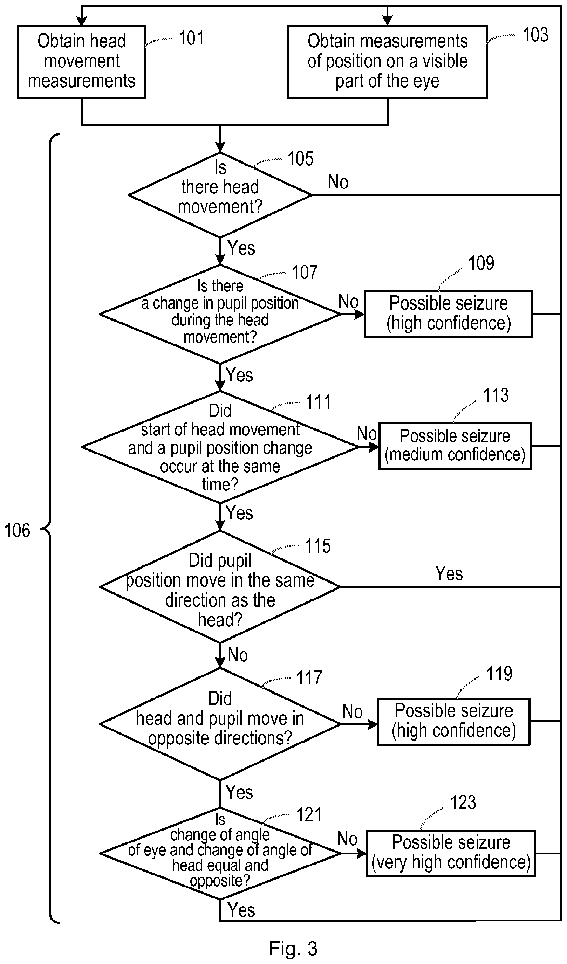

The flow chart in Fig. 3 illustrates an exemplary method according to the techniques described herein for detecting an epileptic seizure in a subject. One or more of the steps of the method can be performed by the processing unit 4 in the apparatus 2, in conjunction with any of the memory unit 6, interface circuitry 8, user interface 10, head movement sensor 12 and pupil position sensor 14, as appropriate. The processing unit 4 may perform the one or more steps in response to executing computer program code, that can be stored on a computer readable medium, such as, for example, the memory unit 6.

The method in Fig. 3 can be used to detect seizures in real time (i.e. as they are happening) or near real time (e.g. within a few seconds or minutes), or they can be used to determine whether the subject experienced seizures during a particular (earlier) period of time.

In a first step, step 101, measurements of the movements of the head of the subject are obtained. This step can comprise actively measuring the head movements using the head movement sensor 12 and obtaining a measurement signal, or retrieving previously-obtained measurements from a memory, e.g. from memory unit 6. Depending on the type of sensor used to measure the head movements, step 101 may comprise performing some processing on the measurements (e.g. an acceleration signal or video sequence) to extract the head movement information. Depending on whether the method in Fig. 3 is to detect seizures in real-time, near real-time or for some preceding time period, step 101 can comprise repeatedly obtaining measurements relating to the current head movements of the subject, the recent head movements of the subject or the head movements during a particular period of time.

In a second step, step 103, measurements of the pupil position in at least one eye of the subject are obtained. This step can comprise actively measuring the pupil position using the pupil position sensor 14 and obtaining a measurement signal, or retrieving previously-obtained measurements from a memory, e.g. from memory unit 6. Depending on the type of sensor used to measure the pupil position, step 103 may comprise performing some processing on the measurements (e.g. a video sequence) to extract the pupil position information. As with step 101, depending on whether the method in Fig. 3 is to detect seizures in real-time, near real-time or for some preceding time period, step 103 can comprise repeatedly obtaining measurements relating to the current pupil position of the subject, the recent pupil positions of the subject or the pupil positions during a particular period of time. As such, step 101 and step 103 may occur at the same time.

According to the techniques described herein, the head movements and pupil positions are analysed to identify typical patterns or combinations of movements and pupil positions that occur during a seizure (in order to detect a seizure) and that occur normally (i.e. when a seizure is not occurring). As described in more detail below, the seizure detection is based on analysing the pupil position over time when the head of the subject is moving.

A seizure can last for several seconds or even several minutes, and these patterns or combinations may only be detectable over the duration of the seizure. As such, in the subsequent steps of Fig. 3, a subset of the measurements obtained in steps 101 and 103 relating to a particular time period as defined by a time window are analysed or processed to determine if a seizure was occurring during that time window. The time window preferably has a duration sufficient to span the occurrence of a seizure. For example, if a seizure typically only lasts for 1 minute, a time window can be used that has a length of 1.2 minutes (for example). In the case of real-time or near real-time detection of seizures, the time window is used to select the most recent measurements of head movements and pupil positions covering the length of the time window. Once that subset of measurements has been evaluated, the time window can be moved or shifted to include more recent measurements, with older measurements falling out of the time window. In the case of the analysis of a previously-obtained set of measurements, the time window can be used to select a subset of the measurements to determine whether a seizure occurred in that time period, and the time window can be moved or shifted along the measurements, and the measurements in the shifted window evaluated to determine if a seizure occurred in that time period.

As noted above, the seizure detection is based on analysing the pupil position over time when the head of the subject is moving. Thus, in step 105, it is determined whether the measurements of the head movements indicate that the subject's head is moving. Step 105 relates to a subset of the head movement measurements (i.e. for a time period relating to a time window), and thus in step 105 it can be determined whether there is any movement of the head of the subject during that time period. Step 105 can comprise comparing the magnitude of the head movements in the head movement measurements to a threshold value, and determining that there is movement of the head of the subject if the magnitude exceeds the threshold value, or determining that there is movement of the head of the subject if the magnitude exceeds the threshold value for at least a minimum period of time (e.g. one second). In another implementation, step 105 can comprise double integrating the movement measurements according to a moving window. For example, using a window width of 1 second, it is assumed that the head has zero velocity at the start of the window (this value can be refined using the computed velocity at that moment in time from the preceding windows), and the movement measurements (e.g. acceleration) are double integrated (compensating for gravity) to determine the displacement at the end of the window (i.e. after 1 second, in this example). If this displacement exceeds a threshold value, then it can be determined that the head is moving. In another implementation of step 105, the acceleration-based head movement measurements can be summed or integrated between zero crossings of the head accelerations (after compensating for gravity), and the result of the sum can be compared to a threshold value. If the threshold value is exceeded, then head movement is detected. In the case of a rotation or orientation sensor (e.g. a gyroscope), step 105 can comprise determining that the head is moving if the amount of rotation or change in orientation exceeds a threshold value.

If no head movements (or insufficient head movements) are occurring within the time period, then the method is not able to determine if a seizure is occurring and the method can return to steps 101 and 103 so further measurements can be obtained (in the real-time/near real-time case), or the time window can be shifted along the measurements to select a new subset of measurements. In either case, when a new subset of measurements is available, step 105 is repeated for those measurements.

If there are head movements at at least some point in the time period, then the measurements of the head movements and the measurements of the pupil position for the time period are processed in step 106 to determine whether the subject experienced a seizure in the time period.

As noted below, in step 106 seizures can be detected by testing the measurements of the head movements and the measurements of the pupil position for various patterns or characteristics, and, depending on the particular patterns or characteristics that are detected in the measurements, seizures can be detected with varying levels of confidence. Fig. 3 sets out four 'tests' of the head movement measurements and the measurements of the pupil position within step 106 (steps 107-121) that can be used to detect seizures with different levels of confidence (or determine that no seizure occurred in that time period). However, it will be appreciated that it is not necessary for step 106 to be implemented with all four tests, and a practical implementation can make use of any one or more of the illustrated tests (i.e. a practical implementation can include a subset of steps 107-121).

In a first test/step of step 106, it is determined in step 107 whether there is/was a change in pupil position at the time that head movement occurred. For example, for any part of the time period where head movement occurred, it is determined from the pupil position measurements whether there was a change in pupil position. Where discrete pupil positions are identified, a change in pupil position may be where there is a change in the discrete pupil position, e.g. from P2 to P1 (as shown in Fig. 2). Where the pupil position is measured and represented more precisely (e.g. as a distance or angle), a change in pupil position may be where the position changes greater than a threshold amount (e.g. there is a distance movement greater than a threshold or an angular displacement greater than a threshold).

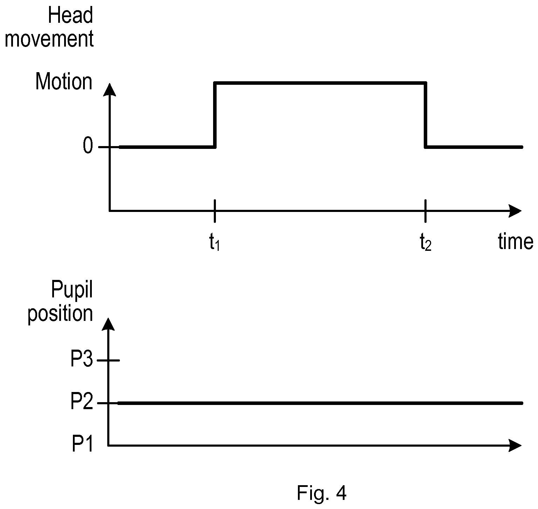

If it is determined that there was no change in pupil position when head movements were occurring, then this is an indication that a seizure may have occurred (as indicated by box 109). When a person (that is not experiencing a seizure) moves their head (e.g. turns to the left), the eyes tend to move in the head to focus on different objects/elements/features in the environment as the head moves/turns. When a subject is experiencing a seizure, the subject may not be focussing (or be able to focus) on any feature in the environment, and this pupil movement may not occur as the head moves. This combination of head movement and lack of pupil movement is a reasonably reliable indicator of a seizure (since it is almost impossible for the head to move without the eye moving relative to the head), and thus a seizure detected by the negative outcome of step 107 is deemed to have a high confidence. This combination of head movement but no pupil movement for a subject is illustrated in Fig. 4. The top graph in Fig. 4 shows the head movement/motion over time (where a value of zero represents no movement, and a non-zero value represents movement), and the bottom graph shows the pupil position over time. It can be seen in the top graph that there is some head movement between times t₁ and t₂, but it can also be seen in the bottom graph that the pupil is in position P2 (i.e. centred) before, during and after the head movement, so there is no pupil movement at the same time as the head movement, which provides a high-confidence indication of a seizure.

After detecting a possible seizure in step 109, the method returns to steps 101/103 (or step 105) and repeats for a new subset of measurements.

It will be appreciated that if there are different head movements at several points during the time period (which are identified in step 105), then step 107 can comprise determining if there was a change in pupil position during each separate head movement. In that case, step 107 can provide a respective decision outcome for each separate head movement (as each head movement could be during a seizure).

If it is determined in step 107 that there was a change in pupil position during the head movement, then a seizure has not yet been detected (and indeed no seizure may have occurred). The method can then pass to step 111, in which it is determined whether a pupil position change started at the same time as the head movement.

If it is determined that the change in pupil position did not occur at the same time that a head movement started, then this is an indication that a seizure may have occurred (as indicated by box 113). In practical terms, when a person (that is not experiencing a seizure) moves their head (e.g. turns to the left), the eyes tend to move at the same time that the head movement starts to either focus on different objects/elements/features in the environment in the direction that the head is turning (so the eye is 'looking ahead' as the head turns), or the gaze of the subject remains fixed on an object/element/feature in the environment as the head moves/turns (which means that the pupil position moves in an opposite direction to the direction in which the head turns, e.g. when the head turns to the left, the pupil moves to the right to maintain the subject's gaze on an object). When a subject is experiencing a seizure, this change in pupil position the subject may not occur when the head starts to move. This combination of head movement and lack of pupil movement at the start of the head movement provides a slightly less reliable indicator of a seizure compared to the 'no' outcome of step 107, and thus a seizure detected by the negative outcome of step 111 is deemed to have a medium confidence.

After detecting a possible seizure in step 113, the method returns to steps 101/103 (or step 105) and repeats for a new subset of measurements.

It will be appreciated that if there are different head movements at several points during the time period (which are identified in step 105) during which a pupil position change occurred (as determined in step 107), then step 111 can comprise determining if there was a change in pupil position at the start of each separate head movement. In that case, step 111 can provide a respective decision outcome for each separate head movement (as each head movement could be a seizure).

It will be appreciated that in step 111 it may not be required to detect a pupil position change at precisely the same time that a head movement started, so some tolerance between the start time of the head movement and the occurrence of a pupil position change can be permitted (for example the pupil position change can be detected to be at the same time as the start of the head movement if the pupil position change occurred within a threshold amount of time to the start of a head movement, such as within 10's or 100's of milliseconds, ms, e.g. 100ms).

If it is determined in step 111 that there was a change in pupil position at the time that the head movement started, then a seizure has not yet been detected (and indeed no seizure may have occurred). The method can then pass to step 115, in which it is determined whether the pupil position change at the start of the head movement was in the same direction as the head movement (for example, if the head movement was a turn to the left, did the pupil position change to the left at the start of that head turn?).

The direction in which the head has moved can be determined from the head movement measurements (e.g. by analysis of the orientation of the head over time), and likewise the direction in which the pupil position has changed can be determined from the pupil position measurements. Where the direction of movement of the head and direction of pupil position change is measured relatively precisely (e.g. in terms of a number of degrees), step 115 can determine that the head movement was in the 'same' direction as the pupil position change at the start of the head movement is they are within a threshold value of each other (e.g. within 20°, or within 10°).

In an alternative implementation of step 115, step 115 can comprise determining an average direction of head movement over a time window (e.g. 0.1 seconds, 0.2s, 0.5s or Is) and an average direction of pupil position change over the time window, and comparing the average directions. The average direction could be given by the difference between the position at the start and end of the window.

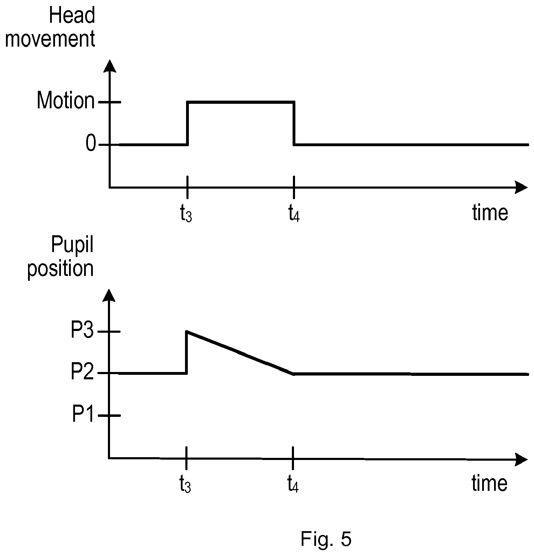

If it is determined that the change in pupil position at the start of the head movement was in the same direction as the head movement, then this is an indication that a seizure has not occurred, and the method returns to steps 101/103 (or step 105) and repeats for a new subset of measurements. This would occur in the 'looking ahead' example above, i.e. where the eyes tend to move to focus on different objects/elements/features in the environment ahead of the direction that the head is turning. Fig. 5 illustrates head movements and pupil positions for a subject that turns their head to look at another object in the environment. The top graph in Fig. 5 shows the head movement/motion over time (where a value of zero represents no movement, and a non-zero value represents movement), and the bottom graph shows the pupil position over time. It can be seen in the top graph that there is some head movement between times t₃ and t₄, for example as the subject turns their head to the left. In the bottom graph the pupil is in position P2 (centred) before the head movement (so before t₃), and the pupil 'flicks' to position P3 (looking to the left) at t₃. Once the head turn to the left starts at t₃, the pupil position slowly moves to the right, back towards P2, which has the effect of the eye gaze being fixed on the new object of interest. The pupil reaches the centred position (P2) at time t₄, the time that the head movement ceases. Thus, according to the method in Fig. 3, the exemplary head movements and pupil positions in Fig. 5 would not result in a seizure being detected as in step 115 it would be determined that the pupil position moved in the same direction as the head (i.e. both to the left at time t₃).

If it is determined in step 115 that the change in pupil position at the start of the head movement was not in the same direction, then it is not yet possible to determine if a seizure has occurred, and the method passes to step 117 where it is determined whether the change in pupil position at the start of the head movement was in the opposite direction to the head movement. For example, if the head movement is a turn to the left, step 117 determines whether the pupil position change is to the right (as opposed to, for example, a pupil position change upwards or downwards). As with step 115, where the direction of movement of the head and direction of pupil position change is measured relatively precisely (e.g. in terms of a number of degrees), step 117 can determine that the head movement was in the 'opposite' direction as the pupil position change at the start of the head movement is they are within a threshold value of being 180° apart (e.g. within 20° of being 180° apart, or within 10° of being 180° apart).

If it is determined in step 117 that the change in pupil position at the start of the head movement was not in the opposite direction to the head movement (and it is known from step 115 that the change was not in the same direction as the head movement), then this is an indication that a seizure may have occurred (as indicated by box 119). Since this step is reached in the event of a particularly unusual combination of head movements and eye movements, there is a high confidence that a seizure has occurred.

After detecting a possible seizure in step 119, the method returns to steps 101/103 (or step 105) and repeats for a new subset of measurements.

If it is determined in step 117 that the change in pupil position at the start of the head movement was in the opposite direction to the head movement, then a seizure has not yet been detected (and indeed no seizure may have occurred). The method can then pass to step 121.

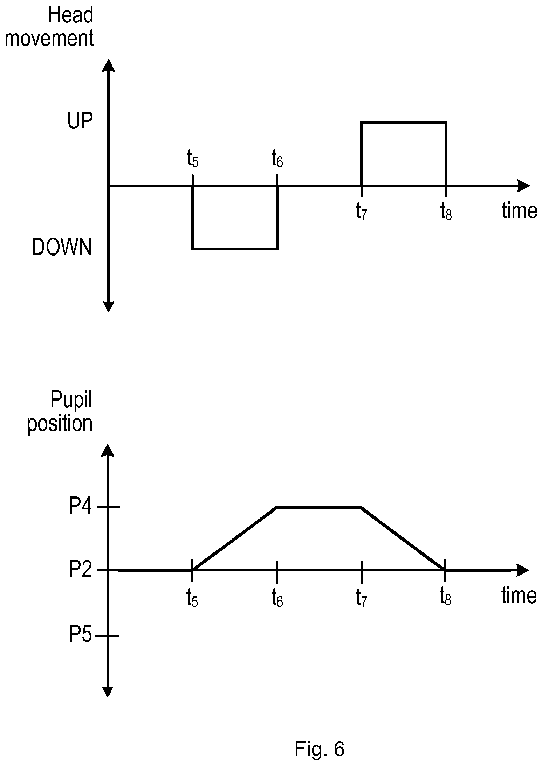

In step 121, it is determined if the change in angle of the pupil in the eye is equal and opposite to the change in angle of the head. For example, when a subject is engaging in conversation with another person and nods their head while maintaining eye contact with the other person, then the change in the angle of the pupil in the eye is equal and opposite to the change in the angle of the head (i.e. the head nods downwards by an amount and the pupil moves upwards by the same amount to maintain eye contact). This is illustrated in Fig. 6. Fig. 6 illustrates head movements and pupil positions for a subject that nods their head while maintaining eye contact. The top graph in Fig. 5 shows the head movement/motion over time (where a value of zero represents no movement, a positive value represents upward movement - i.e. nodding the head up, and a negative value represents downward movement - i.e. nodding the head down). It can be seen in the top graph that there is some head movement downwards between times t₅ and t₆, the head is briefly at rest between t₆ and t₇, and the moves upwards between t₇ and t₈ back to the initial position. The bottom graph shows the pupil position over time, and initially the pupil is in position P2 (centred) before the head movement (so before ts), and the pupil gradually moves to position P4 (to maintain eye contact as the head nods) at t₆ when the head movement stops. From t₇ to t₈ the pupil then gradually moves from position P4 back to P2 (to maintain eye contact as the head moves up to return to the initial position). It will be appreciated that the exact change in the angle of the eye relative to the change in the angle of the head will depend on the focus point, i.e. the distance to the object the subject is looking at. When in 'infinity' (the focus point is a large distance away) the changes in angles are more or less equal. When the focus point is near to the subject, the change in angle of one of the subject's eyes will be larger than the change in the head angle, and the change in angle of the other one of the subject's eyes will be smaller than the change in head angle. These small differences in the change in angles can be accounted for by permitting some tolerance on one or both of the requirements of 'equal' and 'opposite' in step 121.

If in step 121 it is determined that the change in angle of the pupil in the eye is equal and opposite to the change in angle of the head (e.g. as in Fig. 6), then no seizure has occurred and the method can return to steps 101/103 (or step 105) and repeat for a new subset of measurements.

If it is determined in step 121 that the change in angle of the pupil was not equal and opposite to the change in angle of the head, then this is an indication that a seizure may have occurred (as indicated by box 123). Since this step is reached in the event of a particularly unusual combination of head movements and eye movements, there can be a very high confidence that a seizure has occurred. The method can return to steps 101/103 (or step 105) and repeat for a new subset of measurements.

It will be appreciated that where the measurements are obtained of the pupil position for both eyes of the subject, both sets of pupil position measurements can be processed to detect if a seizure has occurred. In some embodiments, the measurements of pupil position for each eye can be analysed individually (e.g. the flow chart in Fig. 3 can be performed separately on each set of measurements). In other embodiments, the two sets of pupil position measurements can be combined (e.g. by taking an average of the pupil position at each sampling instant), and then the averaged set of pupil position measurements can be analysed using the flow chart in Fig. 3. In other embodiments, the two sets of pupil position measurements can be analysed to determine a set of coherence measures indicating the coherence between the pupil positions in each eye over the time period, and this set of coherence measures can be analysed using the flow chart in Fig. 3. The coherence measure can be determined by computing the cross correlation between the two sets of pupil position measurements over time. Optionally, the coherence measurement can be analysed to determine if a seizure is occurring, even if head movements are not present. In particular, if the coherence measure indicates that the eyes are not moving coherently (e.g. the eyes are not looking in a consistent manner at an object), then this can be an indication that a seizure is occurring.

There is therefore provided an efficient and relatively unobtrusive technique for detecting an epileptic seizure in a subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for detecting an epileptic seizure in a subject, the apparatus comprising a processing unit configured to:
obtain head movement measurements, wherein the head movement measurements are measurements of movements of a head of the subject;
obtain pupil position measurements, wherein the pupil position measurements are measurements of a position of a pupil in a visible part of an eye of the subject;
process the head movement measurements relating to a time period to determine if the head moved in the time period; and
if the head moved in the time period, process the head movement measurements and the pupil position measurements for the time period to determine whether the subject experienced a seizure in the time period.

2. An apparatus as claimed in claim 1, wherein the processing unit is configured to:
process the head movement measurements and the pupil position measurements for the time period to determine if there was a change in pupil position at a time that a head movement occurred; and
detect a seizure if there was no change in pupil position at a time that a head movement occurred.

3. An apparatus as claimed in claim 1 or 2, wherein the processing unit is configured to:
process the head movement measurements and the pupil position measurements for the time period to determine if a change in pupil position occurred at the same time that a head movement started; and
detect a seizure if there was no change in pupil position at the same time that a head movement started.

4. An apparatus as claimed in any of claims 1-3, wherein the processing unit is configured to:
process the head movement measurements and the pupil position measurements for the time period to determine if a direction in which the pupil position changed is the same direction as the head movement; and
determine that no seizure has occurred if the direction in which the pupil position changed is the same direction as the head movement.

5. An apparatus as claimed in claim 4, wherein the processing unit is further configured to:
if the direction in which the pupil position changed is not the same direction as the head movement, process the head movement measurements and the pupil position measurements for the time period to determine if the direction in which the pupil position changed is the opposite direction to the head movement; and
determine that a seizure has occurred if the direction in which the pupil position changed is not the same direction as the head movement or opposite to the direction of the head movement.

6. An apparatus as claimed in claim 5, wherein the processing unit is further configured to:
if the direction in which the pupil position changed is opposite to the direction of head movement, process the head movement measurements for the time period to determine changes of angle of the head over the time period;
process the pupil position measurements for the time period to determine changes of angle of the pupil over the time period;
process the changes of angle of the head and changes of angle of the pupil to determine if the changes of angle of the head are equal and opposite to the changes of angle of the pupil;
determine that a seizure has occurred if the changes of angle of the head are not equal and opposite to the changes of angle of the pupil; and
determine that a seizure has not occurred if the changes of angle of the head are equal and opposite to the changes of angle of the pupil.

7. An apparatus as claimed in any of claims 1-6, wherein the apparatus further comprises a head movement sensor for measuring the movements of the head of the subject.

8. An apparatus as claimed in any of claims 1-7, wherein the apparatus further comprises a pupil position sensor for measuring the pupil position in a visible part of an eye of the subject.

9. A method of detecting an epileptic seizure in a subject, the method comprising:
obtaining (101) head movement measurements, wherein the head movement measurements are measurements of movements of a head of the subject;
obtaining (103) pupil position measurements, wherein the pupil position measurements are measurements of a position of a pupil in a visible part of an eye of the subject;
processing (105) the head movement measurements relating to a time period to determine if the head moved in the time period; and
if the head moved in the time period, processing (107, 109, 111, 113, 115, 117, 119) the head movement measurements and the pupil position measurements for the time period to determine whether the subject experienced a seizure in the time period.

10. A method as claimed in claim 9, wherein the step of processing the head movement measurements and the pupil position measurements comprises:
processing (107) the head movement measurements and the pupil position measurements for the time period to determine if there was a change in pupil position at a time that a head movement occurred; and
detecting (109) a seizure if there was no change in pupil position at a time that a head movement occurred.

11. A method as claimed in claim 9 or 10, wherein the step of processing the head movement measurements and the pupil position measurements comprises:
processing (111) the head movement measurements and the pupil position measurements for the time period to determine if a change in pupil position occurred at the same time that a head movement started; and
detecting (113) a seizure if there was no change in pupil position at the same time that a head movement started.

12. A method as claimed in any of claims 9-11, wherein the step of processing the head movement measurements and the pupil position measurements comprises:
processing (115) the head movement measurements and the pupil position measurements for the time period to determine if a direction in which the pupil position changed is the same direction as the head movement; and
determining that no seizure has occurred if the direction in which the pupil position changed is the same direction as the head movement.

13. A method as claimed in claim 12, wherein the step of processing the head movement measurements and the pupil position measurements comprises:
if the direction in which the pupil position changed is not the same direction as the head movement, processing (117) the head movement measurements and the pupil position measurements for the time period to determine if the direction in which the pupil position changed is the opposite direction to the head movement; and
determining (119) that a seizure has occurred if the direction in which the pupil position changed is not the same direction as the head movement or opposite to the direction of the head movement.

14. A method as claimed in claim 13, wherein the step of processing the head movement measurements and the pupil position measurements further comprises:
if the direction in which the pupil position changed is opposite to the direction of head movement, processing the head movement measurements for the time period to determine changes of angle of the head over the time period;
processing the pupil position measurements for the time period to determine changes of angle of the pupil over the time period;
processing (121) the changes of angle of the head and changes of angle of the pupil to determine if the changes of angle of the head are equal and opposite to the changes of angle of the pupil;
determining (123) that a seizure has occurred if the changes of angle of the head are not equal and opposite to the changes of angle of the pupil; and
determine that a seizure has not occurred if the changes of angle of the head are equal and opposite to the changes of angle of the pupil.

15. A computer program product comprising a computer readable medium having computer readable code embodied therein, the computer readable code being configured such that, on execution by a suitable computer or processor, the computer or processor is caused to perform the method of any of claims 9-14.
